# EUROPEAN PATENT APPLICATION

(11) **EP 1 389 470 A1**
(43) Date of publication of application: **18.02.2004**
(21) Application number: 02425722.2
(22) Date of filing: 25.11.2002
(51) Int. Cl.: A61L 2/18, A61C 1/00, A01N 35/04

(54) **Use of active solution for disinfecting and/or sterilizing a dental unit and method thereof**

(30) Priority: 05.08.2002 IT MI20021771
(71) Applicant: CASTELLINI S.p.A., I-40013 Castel Maggiore (Bologna) (IT)
(72) Inventor: Castellini, Franco, 40124 Bologna (IT)
(74) Representative: Tansini, Elio Fabrizio

(57) **Abstract**

The present invention relates to the use of active solutions for disinfecting and/or sterilizing a dental unit. Moreover, the present invention relates to a method for disinfecting and/or sterilizing the hydraulic circuit within a dental unit by using active solutions.

## Description

The present invention relates to the use of active solutions for disinfecting and/or sterilizing a dental unit. Moreover, the present invention relates to a method for disinfecting and/or sterilizing the hydraulic circuit within a dental unit by using active solutions.

In the field of dentistry it is known about the use of oxidizing compounds and/or solutions with a disinfecting activity for cleaning and disinfecting dental units and tools such as handpieces and burs.

The compositions and/or solutions. with biocidal activity that are present on the market are used for disinfecting outer surfaces.

Moreover, it is known about the use of a method for sterilizing in autoclave surgical instruments and handpieces. Autoclave sterilization is a highly efficient method for carrying out a bacterial decontamination of tools and/or parts of a dental unit that can be dismounted and placed in the autoclave.

Autoclave sterilization allows to act both on the outer and on the inner surface.

It is known that a dental unit comprises a so-called handpiece or forceps, which is the terminal end of a series of ducts for flows of compressed air and water and/or mixtures of air and water.

With said handpiece the dentist can carry out a series of operations within the patient's mouth.

A point arranged on the handpiece is cooled by using a flow of water. Said point is mounted onto the handpiece and used for drilling and cutting. The point cooling is carried out by means of a flow of water supplied together with air so as to obtain a mixture, the known spray (vaporized water + compressed air).

During the operations on the patient the dentist introduces into the latter's mouth said handpiece, through which he can carry out a series of operations on the various dental parts. Bacterial contaminations from the handpiece to the patient and from the patient to the handpiece are therefore unavoidable. That is why the problem of hygienic safety plays an important role in the field of dentistry. Such problem obliges dentists and dental technicians to take a series of measures so as to avoid the transmission of contaminations among their patients.

A kind of bacterial contamination that has to be taken into serious consideration is the one occurring when, at the end of a given operation, the dentist stops the spray water + air flow.

In this situation, when the air + water flow is stopped, no water drops from the handpiece. Such an effect takes place because on the one hand there is a pressure of the water within the tubes that would tend to push outside, and on the other hand there is a minimal re-activation of the flow in the opposite direction with respect to the exit so as to prevent its dropping.

The working of a handpiece following the aforesaid scheme prevents water from dropping. but transfers microbes and bacteria from the oral cavity, where pieces of tissue are vaporized, into the handpiece. Microbes and viruses within the patient's oral cavity can therefore get into the tool (handpiece) and contaminate the whole supply duct for water + compressed air and then reach the unit's inner circuit containing water.

Experimental tests have been carried out by using for some days a dental unit with a perfectly sterilized handpiece, equipped with a sterile point and supplied with sterile water taken from a standardized container. At the end of said tests some bacteriological analyses have been carried out on the tank water, which have detected traces of microbial contaminations from the patients to the tank, thus contaminating the whole inner duct, the water circuits of the dental unit and the water tank.

In other words, it happens that microbes and/or viruses spread from the forceps, handpiece, to the ducts and reach the container-bottle.

The transfer of microbes and/or viruses from the handpiece to the whole circuit is helped by concentration gradients ensuring the spreading of microbes into the circuit.

Furthermore, a contribution to the spreading of such microbial masses is due to the movements of liquids within the ducts.

In order to solve the problems concerning bacterial contaminations in dental units a series of operating modes have been adopted, which consist in introducing a disinfecting substance into the water ducts.

To say it otherwise, a disinfectant gets through the water circuits, thus ensuring that all the bacteria that might have moved from the patient into the ducts can be wholly eliminated before operating on the following patient.

Practically, for disinfecting and/or sterilizing the removable parts of the dental unit, such as handpieces, said parts are placed within the device to be used for an autoclave sterilization, whereas for disinfecting the water circuits of the dental unit the following steps are followed.

At given times, for instance the evening before or during a break of the working day, a complete hygienic operation is carried out, i.e. a substance with a high sporicidal power is introduced into the water circuits of the dental unit and left within the ducts. The duration of this kind of operation depends on the decontamination degree to be achieved.

In order to carry out a sterilization and, therefore, reach a sporicidal effect with the substances available on the market a contact time of some hours, on average 4-6 hours, is necessary.

The latter kind of treatment, i.e. sterilization, can therefore be carried out only at the end of the day.

As a matter of fact, carrying out this operation during normal working hours would result in a very long stop for the dental unit, and subsequently in the impossibility to carry out other operations on the patients.

Anyhow, the disinfection and/or sterilization steps described above cannot be carried out when the patient is sitting on the dental unit. The unit cannot be disinfected and/or sterilized if the patient is present.

Moreover, the disinfection and/or sterilization steps cannot be carried out in the interval between the exit of one patient and the arrival of another one.

Disinfection and/or sterilization carried out for instance with glutaraldehyde perfectly sanitizes the hydraulic circuit within a dental unit, but when operations start again said water circuits are contaminated once more.

Moreover, problems arise not only due to the presence of bacteria within the patient's mouth, but also due to the presence of bacteria in network water containing various kinds of microorganisms.

The microorganisms that are present in the water network are water inhabitants. That is way such microorganisms live well in the water circuits of a dental unit, clinging to the duct walls, multiplying and forming a biofilm which cannot be easily removed after a given time. The microorganisms that are present in the supply network of a hydraulic circuit of a dental unit progressively contaminate the whole circuit, and the biofilm grows and gets thicker on the inner surfaces. When the biofilm scales off the surface of the hydraulic duct of the dental unit, said microorganisms that have grown within the unit are released in the water flow.

Tests have been carried out on water samples coming from the water network, which have been used for some days in a dental unit: the result has been that bacterial contaminations of 100,000 microbes/ml of water used have been found.

We can thus summarize as follows the existing ways of contamination of the inner water circuits of a dental unit:
1. direct contamination from patient to unit;
2. contamination from water supply to dental unit; and
3. contamination in compressed air used for handling pneumatic tools and for sprays.

An aim of the present invention is to provide for the use of active solutions with a high biocidal efficiency that allow to disinfect and/or sterilize a dental unit, or at least a part of it, thus protecting the patient's health from risks of contamination.

Another aim of the present invention is to provide for a method for disinfecting and/or sterilizing a water circuit of a dental, unit, or at least a part of it, which is fast, efficient, cheap and safe for the patient's health.

The technical problem underlying the present invention has been to propose a particular selection of active solutions and a method for disinfecting and/or sterilizing the inner surfaces of the water circuits within a dental unit, or at least a part of said surfaces, which can overcome the limitations and drawbacks of the prior art.

This technical problem has been solved by the Applicant, who has defined some particular active solutions containing specific active substances protecting the patient's health from possible risks of bacterial contamination. The use and method according to the present invention have proved to be easy to carry out, cheap and highly efficient.

The object of the present invention is the use of active solutions for disinfecting and/or sterilizing a water circuit, or a part of it, within a dental unit, whose characteristics are listed in the enclosed independent claims.

Preferred embodiments concerning particular uses of said active solutions are listed in the enclosed dependent claims.

Another object of the present invention is a method for disinfecting and/or sterilizing a water circuit, or at least a part of it, within a dental unit, whose characteristics are listed in the enclosed independent claims.

Other preferred embodiments are described in the enclosed dependent claims.

Further technical features and the advantages of the invention will be more evident from the following detailed description.

Examples of active solutions used in the present invention are:

### Active solution no. 1

Aqueous or hydroalcoholic active solution comprising the active substance ortho-phtalaldehyde; preferably in a concentration of 0.3 to 3%, expressed as grams of active substance in 100 grams of solution.

### Active solution no. 2

Aqueous or hydroalcoholic active solution comprising the active substance peracetic acid; preferably in a concentration of 0.1 to 2%, expressed as_ grams of active substance in 100 grams of solution. Said active substance can be obtained from:
a. Mixture of acetic acid and hydrogen peroxide, or alternatively;
b. TAED (tetraacetylethylenediamine) and an active or nascent oxygen generator chosen among organic peroxides, such as for instance urea peroxide.

### Active solution no. 3

Aqueous or hydroalcoholic active solution comprising the active substance hydrogen peroxide; preferably in a concentration of 3 to 30%, expressed as grams of active substance in 100 grams of solution.

### Active solution no. 4

Aqueous or hydroalcoholic active solution comprising the active substance as association of glutaraldehyde and phenate; preferably with a concentration of glutaraldehyde 0.5 to 2%, expressed as grams of active substance in 100 grams of solution, and with a concentration of sodium or potassium phenate of 0.5 to 2%, expressed as grams of active substance in 100 grams of active solution.

### Active solution no. 5

Aqueous or hydroalcoholic active solution comprising the active substance active iodine (I₂); preferably in a concentration of 0.2 to 2%, expressed as grams of active substance in 100 grams of solution. Said active substance can be obtained from:
a. An association of elementary iodine, preferably in a concentration of 0.5 to 2.5%, expressed as grams in 100 grams of solution, with sodium or potassium iodide; preferably in a concentration of 0.5 to 2.5%, expressed as grams in 100 grams of solution.
b. Iodine polyvinylpyrrolidone (Iodopovidone); preferably in a concentration of 0.5 to 2.5%, expressed as grams in 100 grams of solution.

### Active solution no. 6

Aqueous or hydroalcoholic active solution comprising the active substance active chlorine (Cl₂); preferably in a concentration of 0.025 to 0.25%, expressed as grams of active substance in 100 grams of solution. Said active substance can be obtained from:
a. Chloramine T (sodium paratoluensulphonchloramide); preferably in a concentration of 0.025 to 0.25%, expressed as grams in 100 grams of solution.
b. Dichloramine (paratoluensulphondichloramide); preferably in a concentration of 0.05 to 0.5%, expressed as grams in 100 grams of solution.
c. Trichloroisocyanuric acid (trichloro-S-triazine-trione); preferably in a concentration of 0.03 to 0.3%, expressed as grams in 100 grams of solution.
d. Sodium or potassium dichloroisocyanurate; preferably in a concentration of 0.05 to 0.5%, expressed as grams in 100 grams of solution.
e. Sodium hypochlorite; preferably in a concentration of 0.5 to 5%, expressed as grams in 100 grams of solution.

### Active solution no. 7

Aqueous or hydroalcoholic active solution comprising the active substance chlorine dioxide (ClO₂); preferably in a concentration of 0.01 to 0.1%, expressed as grams of active substance in 100 grams of solution.

### Active solution no. 8

Aqueous or hydroalcoholic active solution comprising an association of adazone (5,7-diphenyl-1,3-diazoadamantan-6-one); preferably in a concentration of 0.005 to 0.05%, expressed as grams of active substance in 100 grams of solution, with peracetic acid; preferably in a concentration of 0.02 to 0.2%, expressed as grams in 100 grams of solution.

The active solutions according to the present invention have such disinfecting and sterilizing properties as to ensure a fungicidal, bactericidal, sporicidal and virocidal action.

Beyond the active substances listed above, the active solutions can comprise additives chosen among: stabilizing agents, ion-trapping agents; a pH stabilizing buffer system.

In the case of active solutions comprising peracetic ions, said solutions can be stabilized by introducing a suitable agent or stabilizing substance, which can prevent the degradation of hydrogen peroxide and/or peracetic ions. The solution can further contain a suitable trapping agent such as EDTA and a suitable surfactant agent.

Said active solutions can be used in a method for disinfecting and/or sterilizing a dental unit, carried out with the solution at room temperature, in particular for disinfecting and/or sterilizing the inner surfaces of water circuits of dental units.

The method according to the present invention comprises the following steps:
- supply the hydraulic circuit, or part of it, within a dental unit with a disinfecting and/or sterilizing active solution containing, an active substance chosen among: ortho-phtalaldehyde, peracetic acid, hydrogen peroxide, glutaraldehyde, active iodine, active chlorine, chlorine dioxide or adazone/peracetic acid;
- let the active solution introduced into the hydraulic circuit react; and
- rinse the hydraulic circuit with a washing aqueous solution.

Preferably, the active solution is used at a temperature of 30 to 40°C and at a pH stabilized for instance at 7 to 8.

Preferably, the method provides for a break after the introduction of the active solution containing the active substance into the hydraulic circuit, so as to allow the active substances to act within the hydraulic circuit of the dental unit.

The use the active solutions according to the present invention allows to carry out a high-level disinfection of the hydraulic circuit of the dental unit in 5 minutes only, and a highly efficient sterilization can be carried out in 10 minutes only.

The results obtained therefore allow to use the aforesaid disinfecting and/or sterilizing method as a system to be carried out between one patient and the following one during a normal working day, and also to adopt a sanitizing and safety system always between one patient and the following one (intra-patient) and/or at the end of the day.

As a consequence, all the patients during the whole day can have a higher protection against possible cross infections transmitted from other patients.

## Claims

1. Use of an aqueous or hydroalcoholic active solution comprising the active substance ortho-phtalaldehyde for disinfecting and/or sterilizing a water circuit, or at least a part of it, within a dental unit.

2. Use according to claim 1, in which said solution has a concentration of 0.3 to 3%, expressed as grams of active substance in 100 grams of solution.

3. Use of an aqueous or hydroalcoholic active solution comprising the active substance peracetic acid for disinfecting and/or sterilizing a water circuit, or at least a part of it, within a dental unit.

4. Use according to claim 3, in which said solution has a concentration of 0.1 to 2%, expressed as grams of active substance in 100 grams of solution.

5. Use according to claim 4, in which said active substance is obtained from a mixture of acetic acid and hydrogen peroxide or from TAED (tetraacetylethylenediamine) and an active or nascent oxygen generator chosen among organic peroxides, such as urea peroxide.

6. Use of an aqueous or hydroalcoholic active solution comprising the active substance hydrogen peroxide for disinfecting and/or sterilizing a water circuit, or at least a part of it, within a dental unit.

7. Method according to claim 6, in which said solution has a concentration of 3 to 30%, expressed as grams of active substance in 100 grams of solution.

8. Use of an aqueous or hydroalcoholic active solution comprising the active substance as an association of glutaraldehyde and phenate for disinfecting' and/or sterilizing a water circuit, or at least a part of it, within a dental unit.

9. Use according to claim 8, in which said solution has a concentration of glutaraldehyde of 0.5 to 2%, expressed as grams of active substance in 100 grams of solution, and a concentration of sodium or potassium phenate of 0.5 to 2%, expressed as grams of active substance in 100 grams of solution.

10. Use of an aqueous or hydroalcoholic active solution comprising the active substance active iodine (I₂) for disinfecting and/or sterilizing a water circuit, or at least a part of it, within a dental unit.

11. Method according to claim 10, in which said solution has a concentration of 0.2 to 2%, expressed as grams of active substance in 100 grams of solution.

12. Use according to claim 11, in which said active substance is obtained from an association of elementary iodine; preferably in a concentration of 0.5 to 2.5%, expressed as grams in 100 grams of solution, with sodium or potassium iodide; preferably in a concentration of 0.5 to 2.5%, expressed as grams in 100 grams of solution.

13. Use according to claim 11, in which said active substance is obtained from iodine polyvinylpyrrolidone (Iodopovidone); preferably in a concentration of 0.5 to 2.5, expressed as grams in 100 grams of solution.

14. Use of an aqueous or hydroalcoholic active solution comprising the active substance active chlorine (Cl₂) for disinfecting and/or sterilizing a water circuit, or at least a part of it, within a dental unit; preferably in a concentration of 0.025 to 0.25%, expressed as grams in 100 grams of solution.

15. Use according to claim 14, in which said active substance is obtained from:
- Chloramine T (sodium paratoluensulphonchloramide); preferably in a concentration of 0.025 to 0.25%, expressed as grams in 100 grams of solution; or
- Dichloramine (paratoluensulphondichloramide); preferably in a concentration of 0.05 to 0.5%, expressed as grams in 100 grams of solution; or
- Trichloroisocyanuric acid (trichloro-S-triazine-trione); preferably in a concentration of 0.03 to 0.3%, expressed as grams in 100 grams of solution; or
- Sodium or potassium dichloroisocyanurate; preferably in a concentration of 0.05 to 0.5%, expressed as grams in 100 grams of solution; or
- Sodium hypochlorite; preferably in a concentration of 0.5 to 5%, expressed as grams in 100 grams of solution.

16. Use of an aqueous or hydroalcoholic active solution comprising the active substance chlorine dioxide (ClO₂ for disinfecting and/or sterilizing a water circuit, or at least a part of it, within a dental unit.

17. Method according to claim 16, in which said solution has a concentration of 0.01 to 0.1%, expressed as grams of active substance in 100 grams of solution.

18. Use of an aqueous or hydroalcoholic active solution comprising an association of adazone (5,7-diphenyl-1,3-diazoadamantan-6-one); preferably in a concentration of 0.005 to 0.05%, expressed as grams in 100 grams of solution, with peracetic acid; preferably in a concentration of 0.02 to 0.2%, expressed as grams in 100 grams of solution.

19. Method for disinfecting and/or sterilizing a water circuit, or at least a part of it, within a dental unit comprising the following steps:
- supply the hydraulic circuit, or at least a part of it, within a dental unit with a disinfecting and/or sterilizing active solution according to any of the claims 1 to 18;
- let the active solution introduced into the hydraulic circuit react; and
- rinse the hydraulic circuit with a washing aqueous solution.

20. Method according to claim 19, in which said active solution according to any of the claims 1 to 18 introduced into the hydraulic circuit is left therein for 3 to 10 minutes for disinfecting and for 10 to 20 minutes for sterilizing the inner surfaces of the water circuits of a dental unit.
